# EUROPEAN PATENT SPECIFICATION

(11) **EP 2 512 392 B1**
(45) Date of publication and mention of the grant of the patent: **08.01.2020**
(21) Application number: 10837136.0
(22) Date of filing: 18.11.2010
(51) Int. Cl.: G01N 21/3554, G01N 21/35, G01N 21/3577, A61F 13/505, A61F 13/42, A61F 13/84

(54) **INFRARED WETNESS DETECTION SYSTEM FOR AN ABSORBENT ARTICLE**
INFRAROT-NÄSSEERKENNUNGSSYSTEM FÜR EINEN SAUGFÄHIGEN ARTIKEL
SYSTÈME DE DÉTECTION INFRAROUGE D'HUMIDITÉ POUR ÉLÉMENT ABSORBANT

(30) Priority: 14.12.2009 US 636888
(43) Date of publication of application: 24.10.2012
(73) Proprietor: Kimberly-Clark Worldwide, Inc., Neenah, Wisconsin 54956 (US)
(72) Inventor: GAKHAR, Sudhanshu, Neenah WI 54956 (US); RANGANATHAN, Sridhar, Suwanee Georgia 30024 (US); ALES III, Thomas, Michael, Neenah WI 54956 (US); SARKIS, Randall, George, Chaska MN 55318 (US)
(74) Representative: Dehns
(86) International application number: PCT/IB2010/055275
(87) International publication number: WO 2011/073825

(56) References cited:
- JP-A- 2005 013 244
- US-A1- 2003 060 789
- US-A1- 2006 069 360
- US-A1- 2006 174 693
- US-A1- 2008 041 792
- US-A1- 2008 145 947
- US-B2- 6 870 479
- US-B2- 7 049 969

## Description

### BACKGROUND

Absorbent articles such as diapers, training pants, incontinence products, feminine hygiene products, swim undergarments, and the like, conventionally include a liquid permeable body-side liner, a liquid impermeable outer cover, and an absorbent structure. The absorbent structure is typically located between the outer cover and the liner for taking in and retaining liquids (e.g., urine) exuded by the wearer. The absorbent structure can be made of, for instance, superabsorbent particles. Many absorbent articles, especially those sold under the trade name HUGGIES by the Kimberly-Clark Corporation, are so efficient at absorbing liquids that it is sometimes difficult to tell whether or not the absorbent article has been insulted with a body exudate, especially when the absorbent article is being worn by a newborn or other very young wearers. Insult amounts in such wearers tend to be very small. Other wearers might also produce very small insults.

Accordingly, various types of moisture or wetness indicators have been suggested for use in absorbent articles. The wetness indicators include various passive indicators such as indicator strips, printing, or other devices within each absorbent article, requiring a caregiver to pay for the wetness indicator in each absorbent article whether or not the caregiver intends to use the wetness indicator. Wetness indicators can also include alarm devices that are designed to assist parents or attendants in identifying a wet diaper condition early on. The devices can produce an audible, tactile, electromagnetic, or visual signal. Many of these devices rely on electronics, including conductive elements within each absorbent article that can increase the expense of the absorbent article.

In some aspects, for instance, conductive threads or foils have been placed in the absorbent articles that extend from the front of the article to the back of the article. The conductive materials serve as conductive leads for a signaling device and form an open circuit in the article that can be closed when a body fluid, such as urine, closes the circuit.

Incorporating conductive leads into absorbent articles, however, has caused various problems. For example, absorbent articles are typically mass produced on very fast moving machinery. Incorporating conductive leads into an absorbent article at conventional machine speeds has been problematic.

In addition, packaged absorbent articles are typically fed through a metal detector to ensure that there are no foreign objects contained in the package. If the conductive leads are made from or contain a metal, the metal detector can be activated registering a false positive. The incorporation of metallic materials into absorbent articles can also cause problems for those wearing the garments when attempting to pass through security gates that include metal detectors.

In view of the above, a need currently exists for a signaling system for an absorbent article that does not require conductive elements containing metal or other devices to be inserted into the interior of the article.

US2008/0041792A1 discloses a wetness detector system for detecting the presence of wetness due to a liquid leak in a system.

### SUMMARY

The present inventors undertook intensive research and development efforts with respect to improving absorbent articles, particularly in providing a wetness indicator only when desired by a caregiver and without adding to the cost of an absorbent article. A need exists for wetness detection in diapers and incontinence products in general. Technology that can be implemented without altering diaper construction is preferred.

The present invention provides a non-invasive signaling device in accordance with claim 1.

A non-invasive sensor measures infrared reflectance at some depth within an absorbent article. A useful approach is a paired IR generator/detector system that can be attached to an appropriate target zone on the outer cover of the absorbent article.

The present invention also provides a urine collection and detection system in accordance with claim 14.

The present disclosure is generally directed to various signaling systems that are particularly well suited for use in conjunction with absorbent articles. The signaling systems, for instance, can be connected to a signaling device that can be configured to emit a signal, such as an audible, tactile, electromagnetic or visual signal, for indicating to a user that urine is present in the absorbent article. For example, in one aspect, the absorbent article includes a diaper and the signaling device is configured to indicate the presence of urine.

More particularly, the present disclosure is directed to signaling systems for absorbent articles that can detect the presence of urine without having to place or insert conductive elements into the interior of the article. For instance, in one aspect, a sensor is mounted to an exterior surface of the absorbent article that is capable of sensing a change on the interior of the article that indicates the presence of urine, which is an infrared-absorbing fluid. The sensor includes a paired IR generator/detector system. Insulting the absorbent article with urine will create a change in infrared reflectance as some of the infrared is absorbed by the insult. The sensor is placed in communication with a signaling device. Once a change within the interior of the absorbent article is detected, the signaling device can be configured to emit a signal that indicates urine is present in the absorbent article.

The present invention is directed to a noninvasive signaling device for sensing and indicating the presence of urine in an absorbent article, the device including a housing and a paired IR generator/detector disposed within the housing, the paired IR generator/detector adapted to sense a change in infrared light reflectance due to an insult of urine to the absorbent article, the paired IR generator/detector including an infrared-generating light-emitting diode and an infrared-detecting phototransistor.

Other features and aspects of the present disclosure are discussed in greater detail herein.

### BRIEF DESCRIPTION OF THE DRAWINGS

The foregoing and other features and aspects of the present disclosure and the manner of attaining them will become more apparent, and the disclosure itself will be better understood by reference to the following description, appended claims, and accompanying drawings.
Figure 1 is a rear perspective view of an absorbent article;
Figure 2 is a front perspective view of the absorbent article illustrated in Fig. 1 including the non-invasive signalling device of the present invention;
Figure 3 is a plan view of the absorbent article shown in Fig. 1 with the article in an unfastened, unfolded and laid flat condition showing the surface of the article that faces away from the wearer;
Figure 4 is a plan view similar to Fig. 3 showing the surface of the absorbent article that faces the wearer when worn and with portions cut away to show underlying features;
Figure 5 is a block diagram of a signaling device of the present disclosure; and
Figure 6 is the sensor transient response to repeated insults using the signaling device and absorbent article of the present disclosure.

Repeat use of reference characters in the present specification and drawings is intended to represent the same or analogous features or elements of the present disclosure.

### DETAILED DESCRIPTION

The present disclosure is generally directed to signaling systems for absorbent articles that indicate to a user when urine has insulted the article. For example, in one aspect, the signaling system is designed to emit a signal when urine is detected in the absorbent article. The absorbent article can be, for instance, a diaper, a training pant, an incontinence product, and the like.

Of particular advantage, signaling systems made in accordance with the present disclosure can sense the presence of urine within the absorbent article without having to construct the absorbent article with any elements or sensors contained in the interior of the article. In the past, for instance, metallic conductive leads were typically placed within the interior of the absorbent article. The signaling systems of the present disclosure, on the other hand, can sense the presence of urine from an exterior surface of the article that can greatly simplify the incorporation of the signaling system into the article.

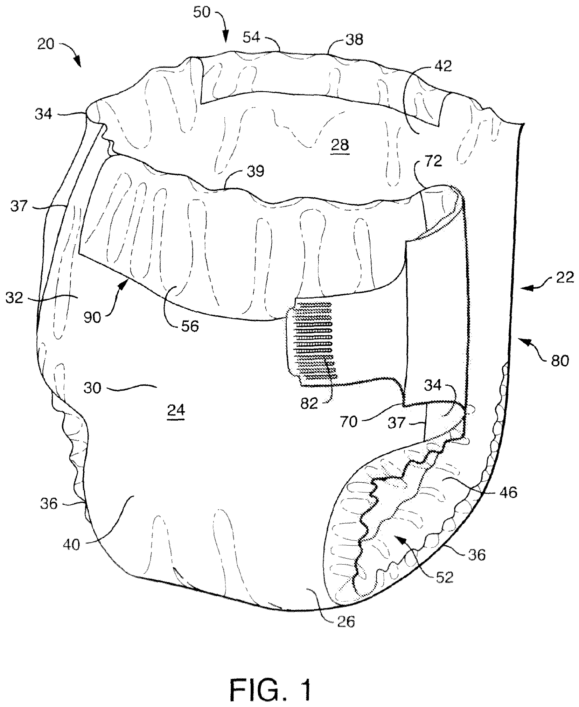

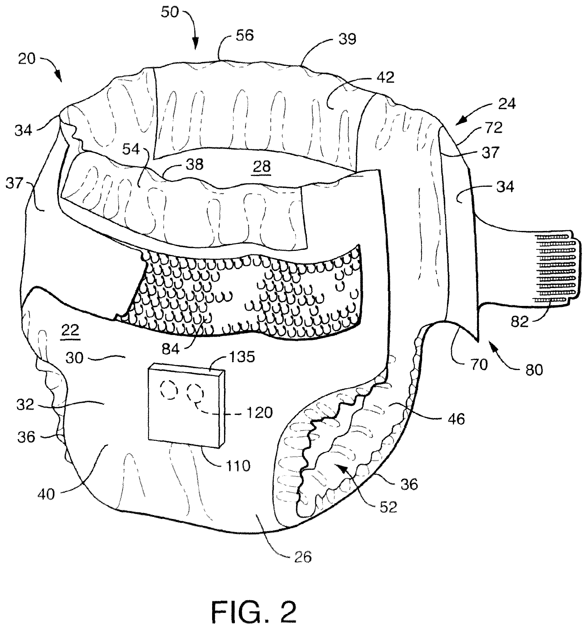

In accordance with the present disclosure, the signaling system can have various configurations and designs. Referring to **Figs. 1** and **2****,** for exemplary purposes, an absorbent article **20** that can be used in conjunction with signaling systems of the present disclosure is shown. The absorbent article **20** can be disposable or not. It is understood that the present disclosure is suitable for use with various other absorbent articles intended for personal wear, including but not limited to diapers, training pants, swim pants, incontinence products, and the like without departing from the scope of the present disclosure.

By way of illustration only, various materials and methods for constructing absorbent articles such as the diaper **20** of the various aspects of the present disclosure are disclosed in PCT Patent Application WO 00/37009 published June 29, 2000 by A. Fletcher et al; U.S. Patent 4,940,464 issued July 10, 1990 to Van Gompel et al.; U.S. Patent 5,766,389 issued June 16, 1998 to Brandon et al., and U.S. Patent 6,645,190 issued November 11, 2003 to Olson et al..

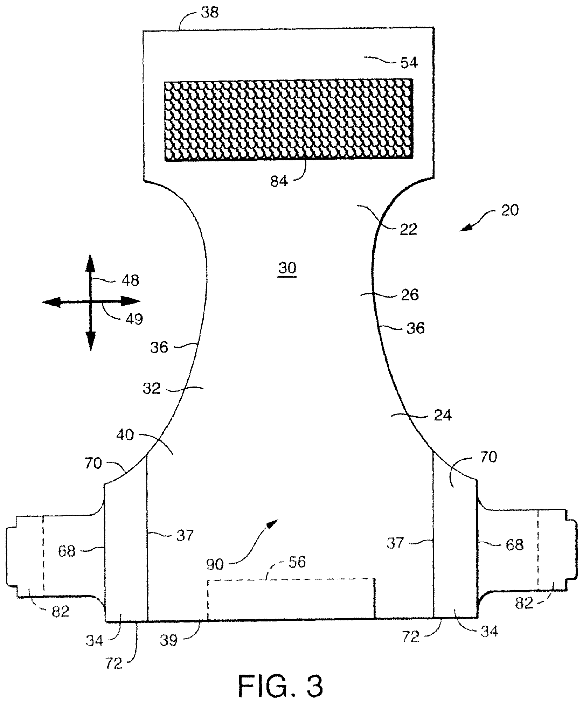

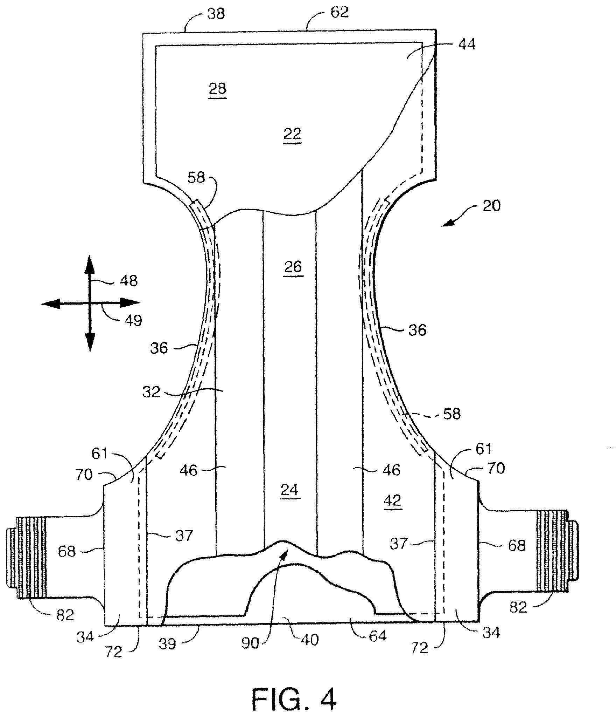

A diaper **20** is representatively illustrated in **Fig. 1** in a partially fastened condition. The diaper **20** shown in **Figs. 1** and **2** is also represented in **Figs. 3** and **4** in an opened and unfolded state. Specifically, **Fig. 3** is a plan view illustrating the exterior side of the diaper **20,** while **Fig. 4** illustrates the interior side of the diaper **20.** As shown in **Figs. 3** and **4****,** the diaper **20** defines a longitudinal direction **48** that extends from the front of the article when worn to the back of the article. Orthogonal to the longitudinal direction **48** is a lateral direction **49.**

The diaper **20** defines a pair of longitudinal end regions, otherwise referred to herein as a front region **22** and a back region **24,** and a center region, otherwise referred to herein as a crotch region **26,** extending longitudinally between and interconnecting the front and back regions **22, 24.** The diaper **20** also defines an inner surface **28** adapted in use (e.g., positioned relative to the other components of the article **20**) to be disposed toward the wearer, and an outer surface **30** opposite the inner surface. The front and back regions **22, 24** are those portions of the diaper **20,** which, when worn, wholly or partially cover or encircle the waist or mid-lower torso of the wearer. The crotch region **26** generally is that portion of the diaper **20** which, when worn, is positioned between the legs of the wearer and covers the lower torso and crotch of the wearer. The absorbent article **20** has a pair of laterally opposite side edges **36** and a pair of longitudinally opposite waist edges, respectively designated front waist edge **38** and back waist edge **39.**

The illustrated diaper **20** includes a chassis **32** that, in this aspect, encompasses the front region **22,** the back region **24,** and the crotch region **26.** Referring to **Figs. 1-4****,** the chassis **32** includes an outer cover **40** and a bodyside liner **42 (****Figs. 1** and **4****)** that can be joined to the outer cover **40** in a superimposed relation therewith by adhesives, ultrasonic bonds, thermal bonds or other conventional techniques. Referring to **Fig. 4****,** the liner **42** can suitably be joined to the outer cover **40** along the perimeter of the chassis **32** to form a front waist seam **62** and a back waist seam **64.** As shown in **Fig. 4****,** the liner **42** can suitably be joined to the outer cover **40** to form a pair of side seams **61** in the front region **22** and the back region **24.** The liner **42** can be generally adapted, i.e., positioned relative to the other components of the article **20,** to be disposed toward the wearer's skin during wear of the absorbent article. The chassis **32** can further include an absorbent structure **44** particularly shown in **Fig. 4** disposed between the outer cover **40** and the bodyside liner **42** for absorbing liquid body exudates exuded by the wearer, and can further include a pair of containment flaps **46** secured to the bodyside liner **42** for inhibiting the lateral flow of body exudates.

The elasticized containment flaps **46** as shown in **Fig. 4** define a partially unattached edge that assumes an upright configuration in at least the crotch region **26** of the diaper **20** to form a seal against the wearer's body. The containment flaps **46** can extend longitudinally along the entire length of the chassis **32** or can extend only partially along the length of the chassis. Suitable constructions and arrangements for the containment flaps **46** are generally well known to those skilled in the art and are described in U.S. Patent 4,704,116 issued November 3, 1987 to Enloe.

To further enhance containment and/or absorption of body exudates, the diaper **20** can also suitably include leg elastic members **58 (****Fig. 4****),** as are known to those skilled in the art. The leg elastic members **58** can be operatively joined to the outer cover **40** and/or the bodyside liner **42** and positioned in the crotch region **26** of the absorbent article **20.**

The leg elastic members **58** can be formed of any suitable elastic material. As is well known to those skilled in the art, suitable elastic materials include sheets, strands or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic retractive forces are imparted to the substrate. In one particular aspect, for example, the leg elastic members **58** can include a plurality of dry-spun coalesced multifilament spandex elastomeric threads sold under the trade name LYCRA and available from Invista, Wilmington, Delaware, U.S.A.

In some aspects, the absorbent article **20** can further include a surge management layer (not shown) that can be optionally located adjacent the absorbent structure **44** and attached to various components in the article **20** such as the absorbent structure **44** or the bodyside liner **42** by methods known in the art, such as by using an adhesive. A surge management layer helps to decelerate and diffuse surges or gushes of liquid that can be rapidly introduced into the absorbent structure of the article. Desirably, the surge management layer can rapidly accept and temporarily hold the liquid prior to releasing the liquid into the storage or retention portions of the absorbent structure. Examples of suitable surge management layers are described in U.S. Pat. No. 5,486,166; and U.S. Pat. No. 5,490,846. Other suitable surge management materials are described in U.S. Pat. No. 5,820,973.

As shown in **Figs. 1-4****,** the absorbent article **20** further includes a pair of opposing elastic side panels **34** that are attached to the back region of the chassis **32.** As shown particularly in **Figs. 1** and **2****,** the side panels **34** can be stretched around the waist and/or hips of a wearer to secure the garment in place. As shown in **Figs. 3** and **4****,** the elastic side panels are attached to the chassis along a pair of opposing longitudinal edges **37.** The side panels **34** can be attached or bonded to the chassis **32** using any suitable bonding technique. For instance, the side panels **34** can be joined to the chassis by adhesives, ultrasonic bonds, thermal bonds, or other conventional techniques.

In an alternative aspect, the elastic side panels can also be integrally formed with the chassis **32.** For instance, the side panels **34** can include an extension of the bodyside liner **42,** of the outer cover **40,** or of both the bodyside liner **42** and the outer cover **40.**

In the aspects shown in the figures, the side panels **34** are connected to the back region of the absorbent article **20** and extend over the front region of the article when securing the article in place on a user. It should be understood, however, that the side panels **34** can alternatively be connected to the front region of the article **20** and extend over the back region when the article is donned.

With the absorbent article **20** in the fastened position as partially illustrated in **Figs. 1** and **2****,** the elastic side panels **34** can be connected by a fastening system **80** to define a 3-dimensional diaper configuration having a waist opening **50** and a pair of leg openings **52.** The waist opening **50** of the article **20** is defined by the waist edges **38** and **39** that encircle the waist of the wearer.

In the aspects shown in the figures, the side panels are releasably attachable to the front region **22** of the article **20** by the fastening system. It should be understood, however, that in other aspects the side panels can be permanently joined to the chassis **32** at each end. The side panels can be permanently bonded together, for instance, when forming a training pant or absorbent swimwear.

The elastic side panels **34** each have a longitudinal outer edge **68,** a leg end edge **70** disposed toward the longitudinal center of the diaper **20,** and waist end edges **72** disposed toward a longitudinal end of the absorbent article. The leg end edges **70** of the absorbent article **20** can be suitably curved and/or angled relative to the lateral direction **49** to provide a better fit around the wearer's legs. However, it is understood that only one of the leg end edges **70** can be curved or angled, such as the leg end edge of the back region **24,** or alternatively, neither of the leg end edges can be curved or angled, without departing from the scope of the present disclosure. As shown in **Fig. 4****,** the outer edges **68** are generally parallel to the longitudinal direction **48** while the waist end edges **72** are generally parallel to the transverse axis **49.** It should be understood, however, that in other aspects the outer edges **68** and/or the waist edges **72** can be slanted or curved as desired. Ultimately, the side panels **34** are generally aligned with a waist region **90** of the chassis.

The fastening system **80** can include laterally opposite first fastening components **82** adapted for refastenable engagement to corresponding second fastening components **84.** In the aspect shown in the figures, the first fastening component **82** is located on the elastic side panels **34,** while the second fastening component **84** is located on the front region **22** of the chassis **32.** In one aspect, a front or outer surface of each of the fastening components **82, 84** includes a plurality of engaging elements. The engaging elements of the first fastening components **82** are adapted to repeatedly engage and disengage corresponding engaging elements of the second fastening components **84** to releasably secure the article **20** in its three-dimensional configuration.

The fastening components **82, 84** can be any refastenable fasteners suitable for absorbent articles, such as adhesive fasteners, cohesive fasteners, mechanical fasteners, or the like. In particular aspects the fastening components include mechanical fastening elements for improved performance. Suitable mechanical fastening elements can be provided by interlocking geometric shaped materials, such as hooks, loops, bulbs, mushrooms, arrowheads, balls on stems, male and female mating components, buckles, snaps, or the like.

In the illustrated aspect, the first fastening components **82** include hook fasteners and the second fastening components **84** include complementary loop fasteners. Alternatively, the first fastening components **82** can include loop fasteners and the second fastening components **84** can be complementary hook fasteners. In another aspect, the fastening components **82, 84** can be interlocking similar surface fasteners, or adhesive and cohesive fastening elements such as an adhesive fastener and an adhesive-receptive landing zone or material; or the like.

In addition to possibly having elastic side panels, the absorbent article **20** can include various waist elastic members for providing elasticity around the waist opening. For example, as shown in the figures, the absorbent article **20** can include a front waist elastic member **54** and/or a back waist elastic member **56.**

As best illustrated in **Fig. 2****,** a signaling device **110** includes a paired IR generator/detector **120** that is adapted to detect the presence of urine in the absorbent article **20.** The paired IR generator/detector **120** includes an infrared-generating light-emitting diode (LED) and an infrared-detecting photo-transistor.

Optics technology such as that described herein can be used to detect wetness in an absorbent article. Light attenuates in water and is more readily absorbed by a wet article as opposed to a dry one. This absorption of light by water is especially significant in the near-infrared region of 800 to 980 nanometer wavelengths. A paired IR generator/detector **120,** particularly one tuned to this same wavelength of infrared light, can be used for wetness detection and indication in an absorbent article. The paired IR generator/detector **120,** and the components therein, are selected to be tuned with urine.

Light emitted by an infrared-generating LED is reflected off a surface close to the sensor system and is collected by the infrared-detecting phototransistor positioned adjacent to the infrared-generating LED. The electrical current through the infrared-detecting phototransistor is detected by a voltage drop across a sensing resistor and is proportional to the intensity of the received infrared signal.

When the product is dry, most of the infrared light emitted by the infrared-generating LED is reflected off various components of the absorbent article **20** and is collected by the infrared-detecting phototransistor. This can result, for example, in a full 5 volt output. After the absorbent article **20** has received an insult, more infrared from the infrared-generating LED is absorbed by the insulted absorbent article **20** than was absorbed by the absorbent article **20** when the absorbent article **20** was dry. This greater absorption of infrared light results in less reflectance of infrared light from the absorbent article **20.** In some cases, the amount of infrared light from the infrared-generating LED absorbed by the insulted absorbent article **20** can be greater than the amount of infrared light reflected by the insulted absorbent article **20.** The reduced amount of infrared light received by the infrared-detecting phototransistor results in a reduction in voltage drop across the sensing resistor.

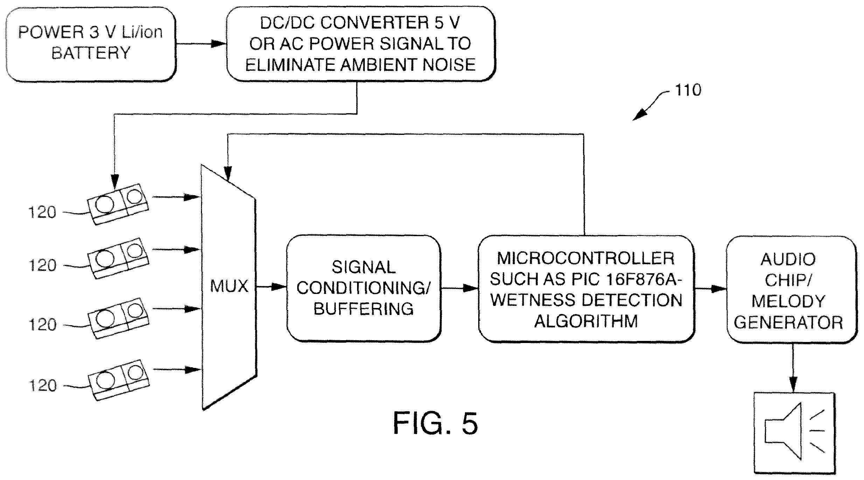

In other aspects of the present disclosure, one or more paired IR generator/detectors **120** can be used in conjunction with the absorbent article **20.** A block diagram of such a system is shown in **Fig. 5****.**

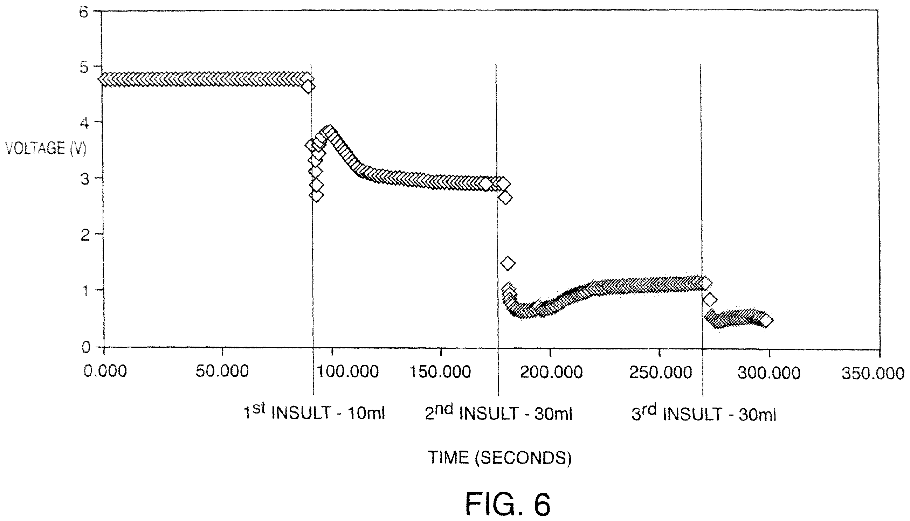

Experimental data proving the concept is shown in **Fig. 6****.** An absorbent article **20** of the present disclosure was laid open and flat on top of a standard circuit using a paired IR generator/detector **120** of the present disclosure. The absorbent article **20** was insulted with 10 ml of saline after 90 seconds, with an additional 30 ml of saline after 180 seconds, and with an additional 30 ml of saline after 270 seconds. The voltage output of the paired IR generator/detector **120** was measured and plotted in **Fig. 6****.** Further experimentation with such a system also indicated a quick response to wetness in the absorbent product (<3 sec) and demonstrated a capability of detecting multiple small voids on the order of 10 ml.

In alternate aspects of the present disclosure, similar sensor arrangements can be used with such sensor arrangements using other infrared wavelengths.

Infrared LEDs are the infrared-generating source because of their low power requirements, allowing for longer life for a given battery or other power source. Suitable paired IR generator/detectors such as an AVAGO Technologies HSDL - 9100 Surface-Mount Proximity Sensor are available from Avago Inc. Additional battery or other power source life can be attained through power use optimization and algorithm development as is known in the art.

Use of the paired IR generator/detector **120** described herein enables a conductor-less wetness sensing solution for absorbent articles that has no impact on current absorbent article manufacturing processes because the paired IR generator/detector is not manufactured as a part of the absorbent article 20, and because the absorbent article 20 has no conductors positioned therein. The paired IR generator/detector has a quick response time, generally less than 3 seconds, is capable of detecting small voids such as those less than 10 milliliters, and can detect multiple insults. The paired IR generator/detector **120** can be sufficiently powered using a commonly-available battery such as a 2032 Li Battery for a battery life of at least three months. Use of the paired IR generator/detector **120** with its common components also allows the device to meet regulatory standards in the United States and in the European Union.

The noninvasive, paired IR generator/detector **120** is used to determine the IR reflectance of material near the sensing element. As described herein, this technology is applied to detect wetness in an absorbent article 20 from outside the outer cover 40. Key challenges, however, with such a paired IR generator/detector **120** are managing the distance of the target from the paired IR generator/detector **120** to have sufficient distance for reflection, and the ability of the system to detect a small amount of wetness from outside the outer cover. Such challenges can be addressed through signal conditioning and algorithms to ignore environmental interferences.

When insult conditions are reached, as determined by the algorithm running the microcontroller such as a PIC 16F876A, an alarm signal is generated as further described herein.

The signaling device **110** can emit any suitable signal to indicate to the user that the article has been insulted. The signal, for instance, can include an audible signal, a tactile signal, an electromagnetic signal, or a visual signal. The audible signal, for instance, can be as simple as a beep or can include a musical tune. In still another aspect, the signaling device can emit a wireless signal that then activates a remote device, such as a telephone or a pager.

Further aspects of the signaling device **110** can be found in co-pending U.S. Patent Application Publication No. US 2010/0164733, entitled "Remote Detection Systems for Absorbent Articles".

The electronics associated with the paired IR generator/detector **120** are relatively simple and can be miniaturized. The complete paired IR generator/detector **120** is disposed in a housing **135** (see **Fig. 2**) that is adapted to be attached to the absorbent article **20,** or held in vicinity to the absorbent article **20.** If the housing **135** is to be attached to the absorbent article **20** using an attachment mechanism, the housing **135** can be a pouch or a rigid or semi-rigid housing **135** that attaches to the outer cover **40** of the absorbent article **20** near the region where insults are expected. Such attachment mechanism can use adhesive, hook and loop, mechanical fasteners such as snaps, a clip, or a clasp, any other suitable attachment mechanism, or any combination of these. Various attachment mechanisms include those disclosed in co-pending and co-assigned U.S. Patent Application Publication No. 2007/0142797 to Long, et al. and entitled "Garments With Easy-To-Use Signaling Device"; U.S. Patent Application Publication No. 2006/0244614 to Long and entitled "Connection Mechanisms"; and U.S. Patent Application Publication No. 2007/0024457 to Long, et al. and entitled "Connection Mechanisms In Absorbent Articles For Body Fluid Signaling Devices".

In another aspect of the present disclosure, the signaling device **110** is adapted to be held near the outermost surface of the outer cover **40** of the absorbent article **20.** In this aspect, no attachment mechanism is needed. The wearer of the absorbent article **20** or a caregiver holds the signaling device **110** near the outer cover **40** of the absorbent article **20** to detect whether the absorbent article **20** has received an insult.

Sensors such as those described herein are further described, for instance, in U.S. Patent Application Publication No. US 2008/0097169 and in U.S. Patent Application Publication No. 2008/0048786.

For example, in one aspect, the system can be configured such that the signaling device will not emit signals within a certain period of time once the system is first activated, where being activated means the system is in a condition to detect and provide a signal. The period of time can vary depending upon the particular circumstances and the particular application. For example, in one aspect, the system can be configured not to emit signals for at least the first 15 minutes, such as at least the first 30 minutes, such as at least the first 45 minutes, such as at least the first hour the absorbent article is worn.

In an alternative aspect, steady state is determined by the paired IR generator/detector **120** used in the system. Steady state can be determined when substantial or significant changes in infrared reflectance fail to occur for a certain period of time indicating that steady state conditions have been reached. For instance, the system can be configured to only become activated once the paired IR generator/detector **120** determines no substantial changes within the interior of the article for a period of about five minutes, such as about 10 minutes, such as about 20 minutes, such as about 30 minutes, such as about 45 minutes, such as about one hour. For example, if the sensor is a paired IR generator/detector **120,** steady state can be determined when the paired IR generator/detector **120** senses no more than about 5 percent change in infrared reflectance on the interior of the article for a period of at least 10 minutes.

When using a paired IR generator/detector **120,** the paired IR generator/detector **120** can be placed in any suitable location on the absorbent article **20.** For instance, the paired IR generator/detector **120** can be placed in the crotch region **26,** on the back region **24,** or on the front region **22** of the article **20** depending upon various factors. As described herein, the paired IR generator/detector **120** is placed on an exterior surface of the outer cover **40** of the absorbent article **20.**

All of the sensors described herein can be configured to be disposed of with the absorbent article **20.**

The paired IR generator/detector **120** can be configured to be removed from the absorbent article **20** when the absorbent article **20** is disposed and placed on a new absorbent article **20.** In fact, in one aspect, the paired IR generator/detector **120** and/or signal device can include multiple settings depending upon the absorbent article **20** to which it is attached. In this manner, the signaling system can be modified based upon the particular product specifications. The product purchased can provide information to the consumer as to which setting to use.

As absorbent articles increase in effectiveness, in one aspect, the signaling system of the present disclosure can be configured to emit a signal or not emit a signal during a first insult of urine and/or to emit a signal when a second insult occurs. In one aspect, for instance, the absorbent article **20** can be constructed so as to be capable of holding two insults of urine from the wearer. A wetness sensing system can be particularly needed for these types of absorbent articles **20** so that a caregiver can differentiate between the first insult and the second insult. In accordance with the present disclosure, the signaling system can be constructed so as to recognize a change within the absorbent article **20** due to the first insult and then readjust the criteria based upon the second insult. Once the second insult is recognized, the signaling system can be designed to emit a signal.

For instance, after a first insult with urine, the paired IR generator/detector **120** can sense a change in infrared light reflectance within the absorbent article **20.** The paired IR generator/detector **120** can also be configured to sense a change in infrared light reflectance after second and succeeding insults as well.

When using a paired IR generator/detector **120** as described herein, in one aspect, the system can be designed to take into account changes in the above measurements when the absorbent article **20** is first placed on the wearer. For example, when the absorbent article **20** is first donned, a change in infrared reflectance can be expected. To account for this change, the system of the present disclosure can be configured to only cause signals to be emitted by the signaling device **110** when steady state conditions within the article have been reached.

In some instances, it is conceivable that the paired IR generator/detector **120** needs to contend with nearby objects that can cause interference. In practical applications, however, such a situation is unlikely because the interference-causing object typically needs to be very close to the paired IR generator/detector **120.** This makes the appearance of an interference-causing object unlikely when the paired IR generator/detector **120** is used in conjunction with an absorbent article **20.** Nevertheless, an interference problem of this sort can be managed by an intelligent algorithm that recognizes and stores signal output once the paired IR generator/detector **120** is in position and activated. The algorithm uses this signal output as a reference point and interprets subsequent signals in relation to this reference point. In other words, the algorithm includes an intelligent zeroing feature.

Once the device is activated, the algorithm takes a baseline measurement, which is automatic and transparent to the user. Once the signaling device **110** is installed by a user, the paired IR generator/detector **120** automatically zeroes itself to establish the point of zero wetness baseline needed.

In another aspect of the present disclosure (not shown), the signaling device **110** uses more than one paired IR generator/detector **120.** For example, two paired IR generator/detectors **120** can be positioned such that one is near the front of the absorbent article **20** to detect urine and the other is near the rear of the absorbent article **20** to detect fecal matter.

## Claims

1. A non-invasive signaling device (110) for sensing and indicating the presence of urine in an absorbent article, the device comprising:
a housing (135); and
a paired IR generator/detector (120) disposed within the housing, the paired IR generator/detector adapted to sense a change in infrared light reflectance due to an insult of urine to the absorbent article, the paired IR generator/detector including an infrared-generating light-emitting diode and an infrared-detecting phototransistor.

2. The device of claim 1, wherein the infrared-generating light-emitting diode generates infrared light with a wavelength of 800 to 980 nanometers.

3. The device of claim 1, wherein the infrared-generating light-emitting diode has been selected to generate a wavelength tuned with urine.

4. The device of claim 1, wherein the housing is flexible.

5. The device of claim 1, wherein the signaling device is configured to become activated only when no substantial changes within the interior of the article occur for a period of five minutes.

6. The device of claim 1, further comprising:
an attachment mechanism for removably attaching the housing to the absorbent article (20); and
wherein the signaling device (110) is adapted to emit a signal when an insult of urine within the absorbent article is sensed by the paired IR generator/detector.

7. The device of claim 6, wherein the signaling device (110) includes a plurality of settings that are selected by the user depending upon at least one specification of the absorbent article.

8. The device of claim 6, wherein the signaling device is configured to differentiate between a first insult of urine of the absorbent article from a second insult of urine of the absorbent article.

9. The device of claim 1, wherein an electrical current through the infrared-detecting phototransistor is detected by a voltage drop across a sensing resistor, and wherein the voltage drop is proportional to the intensity of reflected infrared light.

10. The device of claim 1, wherein the signaling device (110) is adapted to provide notification of the presence of urine in the absorbent article (20).

11. The device of claim 10, wherein the signaling device (110) is adapted to provide a visual notification or an audio notification.

12. The device of claim 10, wherein the signaling device is adapted to provide a wireless notification.

13. The device of claim 10, wherein the signaling device (110) is adapted to provide a tactile notification, wherein the tactile notification is preferably a vibratory notification.

14. A urine collection and detection system comprising:
an absorbent article (20); and
the non-invasive signaling device (110) of claim 1, or any one of claims 10-13.

15. The system of claim 14, wherein the signaling device (110) is adapted to be held adjacent the absorbent article.

## Patentansprüche

1. Nicht invasive Signalisierungsvorrichtung (110) für das Erfassen und Angeben des Vorhandenseins von Urin in einem saugfähigen Artikel, die Vorrichtung umfassend:
ein Gehäuse (135); und
einen gepaarten IR-Generator/Detektor (120), der im Gehäuse angeordnet ist, wobei der gepaarte IR-Generator/Detektor so angepasst ist, dass er eine Änderung bei der Infrarotlicht-Reflektion aufgrund eines Insults von Urin am saugfähigen Artikel erfasst, wobei der gepaarte IR-Generator/Detektor eine infraroterzeugende lichtemittierende Diode und einen infraroterkennenden Phototransistor beinhaltet.

2. Vorrichtung nach Anspruch 1, wobei die infraroterzeugende lichtemittierende Diode Infrarotlicht mit einer Wellenlänge von 800 bis 980 Nanometern erzeugt.

3. Vorrichtung nach Anspruch 1, wobei die infraroterzeugende lichtemittierende Diode so ausgewählt ist, dass sie eine an Urin abgestimmte Wellenlänge erzeugt.

4. Vorrichtung nach Anspruch 1, wobei das Gehäuse flexibel ist.

5. Vorrichtung nach Anspruch 1, wobei die Signalisierungsvorrichtung so konfiguriert ist, dass sie erst aktiviert wird, wenn für einen Zeitraum von fünf Minuten keine wesentlichen Veränderungen im Inneren des Artikels auftreten.

6. Vorrichtung nach Anspruch 1, ferner umfassend:
einen Befestigungsmechanismus für das entfernbare Befestigen des Gehäuses am saugfähigen Artikel (20); und
wobei die Signalisierungsvorrichtung (110) so angepasst ist, dass sie ein Signal abgibt, wenn ein Insult von Urin im saugfähigen Artikel durch den gepaarten IR-Generator/Detektor erfasst wird.

7. Vorrichtung nach Anspruch 6, wobei die Signalisierungsvorrichtung (110) eine Vielzahl von Einstellungen beinhaltet, die durch den Benutzer abhängig von mindestens einer Spezifikation des saugfähigen Artikels ausgewählt ist.

8. Vorrichtung nach Anspruch 6, wobei die Signalisierungsvorrichtung so konfiguriert ist, dass sie zwischen einem ersten Insult von Urin des saugfähigen Artikels und einem zweiten Insult von Urin des saugfähigen Artikels unterscheidet.

9. Vorrichtung nach Anspruch 1, wobei ein elektrischer Strom durch den infraroterkennenden Phototransistor durch einen Spannungsabfall über einen Erfassungswiderstand erkannt wird, und wobei der Spannungsabfall proportional zur Intensität des reflektierten Infrarotlichts ist.

10. Vorrichtung nach Anspruch 1, wobei die Signalisierungsvorrichtung (110) so angepasst ist, dass sie eine Benachrichtigung des Vorhandenseins von Urin im saugfähigen Artikel (20) bereitstellt.

11. Vorrichtung nach Anspruch 10, wobei die Signalisierungsvorrichtung (110) so angepasst ist, dass sie eine visuelle Benachrichtigung oder eine akustische Benachrichtigung bereitstellt.

12. Vorrichtung nach Anspruch 10, wobei die Signalisierungsvorrichtung so angepasst ist, dass sie eine drahtlose Benachrichtigung bereitstellt.

13. Vorrichtung nach Anspruch 10, wobei die Signalisierungsvorrichtung (110) so angepasst ist, dass sie eine taktile Benachrichtigung bereitstellt, wobei die taktile Benachrichtigung bevorzugt eine Vibrationsbenachrichtigung ist.

14. Sammel- und Erkennungssystem für Urin, umfassend:
einen saugfähigen Artikel (20); und
die nicht invasive Signalisierungsvorrichtung (110) nach Anspruch 1 oder irgendeinem der Ansprüche 10-13.

15. System nach Anspruch 14, wobei die Signalisierungsvorrichtung (110) so angepasst ist, dass sie neben dem saugfähigen Artikel gehalten wird.

## Revendications

1. Dispositif de signalement non invasif (110) pour la détection et l'indication de la présence d'urine dans un article absorbant, le dispositif comprenant :
un boîtier (135) ; et
un générateur/détecteur infrarouge apparié (120) disposé dans le boîtier, le générateur/détecteur infrarouge apparié étant adapté pour détecter un changement de réflectance de la lumière infrarouge dû à un écoulement d'urine dans l'article absorbant, le générateur/détecteur infrarouge apparié comprenant une diode électroluminescente générant de l'infrarouge et un phototransistor détectant l'infrarouge.

2. Dispositif selon la revendication 1, dans lequel la diode électroluminescente générant de l'infrarouge génère de la lumière infrarouge à une longueur d'onde de 800 à 980 nanomètres.

3. Dispositif selon la revendication 1, dans lequel la diode électroluminescente générant de l'infrarouge a été choisie pour générer une longueur d'onde syntonisée avec l'urine.

4. Dispositif selon la revendication 1, dans lequel le boîtier est souple.

5. Dispositif selon la revendication 1, dans lequel le dispositif de signalement est configuré pour devenir activé seulement une fois qu'aucun changement sensible à l'intérieur de l'article ne s'est produit pendant une période de cinq minutes.

6. Procédé selon la revendication 1, comprenant en outre :
un mécanisme de fixation destiné à fixer de manière amovible le boîtier à l'article absorbant (20) ; et
dans lequel le dispositif de signalement (110) est adapté pour émettre un signal lorsqu'un écoulement d'urine dans l'article absorbant est détecté par le générateur/détecteur infrarouge apparié.

7. Dispositif selon la revendication 6, dans lequel le dispositif de signalement (110) comprend une pluralité de réglages qui sont choisis par l'utilisateur en fonction d'au moins une spécification de l'article absorbant.

8. Dispositif selon la revendication 6, dans lequel le dispositif de signalement est configuré pour effectuer une différenciation entre un premier écoulement d'urine de l'article absorbant et un second écoulement d'urine de l'article absorbant.

9. Dispositif selon la revendication 1, dans lequel un courant électrique à travers le phototransistor détectant l'infrarouge est détecté par une chute de tension dans une résistance de détection, et dans lequel la chute de tension est proportionnelle à l'intensité de la lumière infrarouge réfléchie.

10. Dispositif selon la revendication 1, dans lequel le dispositif de signalement (110) est adapté pour fournir une notification de la présence d'urine dans l'article absorbant (20) .

11. Dispositif selon la revendication 10, dans lequel le dispositif de signalement (110) est adapté pour fournir une notification visuelle ou une notification sonore.

12. Dispositif selon la revendication 10, dans lequel le dispositif de signalement est adapté pour fournir une notification sans fil.

13. Dispositif selon la revendication 10, dans lequel le dispositif de signalement (110) est adapté pour fournir une notification tactile, dans lequel la notification tactile est de préférence une notification vibratoire.

14. Système de collecte et de détection d'urine comprenant :
un article absorbant (20) ; et
le dispositif de signalement non invasif (110) selon la revendication 1 ou l'une quelconque des revendications 10 à 13.

15. Système selon la revendication 14, dans lequel le dispositif de signalement (110) est adapté pour être maintenu adjacent à l'article absorbant.
